# EUROPEAN PATENT SPECIFICATION

(11) **EP 1 023 449 B1**
(45) Date of publication and mention of the grant of the patent: **16.05.2007**
(21) Application number: 98946554.7
(22) Date of filing: 18.09.1998
(51) Int. Cl.: C12N 15/82, C07K 14/415, C12N 5/10, A01H 5/00

(54) **CONTROL OF PLANT ABSCISSION AND POD DEHISCENCE OR SHATTER**
KONTROLLE DER PFLANZLICHEN ABSZISSION UND SCHOTENDEHISZENZ ODER SCHOTENABWURFS
LUTTE CONTRE L'ABSCISSION DES VEGETAUX ET LA DEHISCENCE OU ECLATEMENT DES GOUSSES

(30) Priority: 19.09.1997 GB 9720038
(43) Date of publication of application: 02.08.2000
(73) Proprietor: BIOGEMMA UK LIMITED, Cambridge CB4 0GZ (GB)
(72) Inventor: PAUL, Wyatt Biogemma UK Limited, Cambridge CB4 4GZ (GB); ROBERTS, Jeremy, Alan University of Nottingham, Loughborough, Leicestershire LE12 5RD (GB); WHITELAW, Catherine University of Nottingham, Loughborough Leicestershire LE12 5RD (GB)
(74) Representative: Flesselles, Bruno F.G.
(86) International application number: PCT/GB1998/002836
(87) International publication number: WO 1999/015680

(56) References cited:
- EP-A- 0 562 836
- WO-A-94/23043
- WO-A-95/13384
- WO-A-96/30529
- XU, W., ET AL. : "arabidopsis TCH4, regulated by hormones and the environment, encodes a xyloglucan endotransglycosylase" THE PLANT CELL, vol. 7, October 1995, pages 1555-1567, XP002090626
- NEWMAN, T., ET AL. : "genes galore: a summary of methods for assessing results from large-scale partial sequencing of anonymous Arabidopsis cDNA clones" EMBL SEQUENCE DATA LIBRARY,30 August 1993, XP002090627 heidelberg, germany
- JENKINS E S ET AL: "MOLECULAR CHARACTERISATION OF BRASSICA NAPUS POD DEHISCENCE" JOURNAL OF EXPERIMENTAL BOTANY, 1 January 1995, page 42 XP000570274
- PARK,J-H., ET AL. : "differential expression of senescence-associated mRNAs during leaf senescence induced by different senescence-inducing factors in Arabidopsis" PLANT MOLECULAR BIOLOGY, vol. 37, June 1998, pages 445-454, XP002090628

### SEQUENCE LISTING

(1) GENERAL INFORMATION:
   (i) APPLICANT:
      (A) NAME: Nickerson Biocem Limited
      (B) STREET: Cambridge Science Park, Milton Road
      (C) CITY: Cambridge
      (E) COUNTRY: Great Britain
      (F) POSTAL CODE (ZIP): CB4 4GZ

      (A) NAME: Wyatt Paul, c/o Nickerson Biocem Limited
      (B) STREET: Cambridge Science Park, Milton Road
      (C) CITY: Cambridge
      (E) COUNTRY: Great Britain
      (F) POSTAL CODE (ZIP): CB4 4GZ

      (A) NAME: Jeremy Alan Roberts, c/o University of Nottingham, Division of Plant Science
      (B) STREET: School of Biological Sciences, Sutton Bonington Campus
      (C) CITY: Loughbourough
      (D) STATE: Leicestershire
      (E) COUNTRY: Great Britain
      (F) POSTAL CODE (ZIP): LE12 5RD

      (A) NAME: Catherine Whitelaw, c/o University of Nottingham, Division of Plant Science
      (B) STREET: School of Biological Sciences, Sutton Bonington Campus
      (C) CITY: Loughbourough
      (D) STATE: Leicestershire
      (E) COUNTRY: Great Britain
      (F) POSTAL CODE (ZIP): LE12 5RD
   (ii) TITLE OF INVENTION: Control
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: WO GB98/02836
(2) INFORMATION FOR SEQ ID NO: 1:
   (i) SEQUENCE CHARACTERISTICS:
      (A) LENGTH: 306 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 1:
   GGTTGGGTCG AACCATAGGT GGAAAGCTTC TTTCTCTCTC GCTTGACAAA TCCTCTGGTT    60
   CGGGTTTTCA GTCCCATCAG GAGTTTCTCT ATGGTAAAGC TGAGGTTCAA ATGAAACTTG   120
   TCCCTGGTAA CTCTGCTGGA ACAGTCACAA CATTCTATCT TAAATCACCG GGAACTACAT   180
   GGGATGAGAT CGATTTCGAG TTCTTGGGAA ACATAAGTGG CCATCCCTAT ACTCTCCATA   240
   CTAATGTTTA CACACGAAGG CTCTGGAGAC AAAGAACAGC AGTTTCATCT ATGGTTCGAC   300
   CCGACC                                                              306
(2) INFORMATION FOR SEQ ID NO: 2:
   (i) SEQUENCE CHARACTERISTICS:
      (A) LENGTH: 101 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 2:
   Leu Gly Arg Thr Ile Gly Gly Lys Leu Leu Ser Leu Ser Leu Asp Lys
   1               5                   10                  15
   Ser Ser Gly Ser Gly Phe Gln Ser His Gln Glu Phe Leu Tyr Gly Lys
               20                  25                  30
   Ala Glu Val Gln Met Lys Leu Val Pro Gly Asn Ser Ala Gly Thr Val
           35                  40                  45
   Thr Thr Phe Tyr Leu Lys Ser Pro Gly Thr Thr Trp Asp Glu Ile Asp
       50                  55                  60
   Phe Glu Phe Leu Gly Asn Ile Ser Gly His Pro Tyr Thr Leu His Thr
   65                  70                  75                  80
   Asn Val Tyr Thr Arg Arg Leu Trp Arg Gln Arg Thr Ala Val Ser Ser
                   85                  90                  95
   Met Val Arg Pro Asp
               100
(2) INFORMATION FOR SEQ ID NO: 3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 3:
      NGTNGGRTCR AACCANAGRT G 21
(2) INFORMATION FOR SEQ ID NO: 4:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 4:
      AGCCAGCGAA 10

## Description

This invention relates generally to the control of plant abscission and pod dehiscence or shatter.

Abscission is the process that causes the shedding of a range of plant parts, including leaves, flowers and fruit. The process occurs at precise sites and involves coordinated cell wall breakdown. Associated with cell separation is an increase in the activity of several hydrolytic enzymes including β-1,4-glucanase (cellulase, EC 3.1.2.4) and polygalacturonase (PG EC 3.2.1.15).

The process of pod dehiscence, or shatter as it is commonly termed, in oilseed rape (*Brassica napus*) and other crops shares a number of features with abscission. Degradation and separation of cell walls occurs along a discrete layer of cells, termed the dehiscence zone, and a localised increase in the activity of cellulase has been reported prior to the onset of dehiscence (Meakin and Roberts *J. Exp. Bot*. 41(229) 995-1002 (1990) and *J. Exp. Bot*. 41(229) 1003-1011 (1990)).

It is understood that the processes of plant abscission and pod dehiscence are not regulated by the same environmental or chemical signals but that both are the consequence of cell wall degradation.

The process of pod dehiscence is agronomically important because it may result in the premature shedding of seed before the crop can be harvested. Adverse weather conditions can exacerbate the process resulting in a greater than 50% loss of seed. This loss of seed not only has a dramatic effect on yield but also results in the emergence of the crop as a weed in the subsequent growing season.

Attempts to solve this problem over the last 20 years have focused on the breeding of shatter-resistant varieties. The most commonly used method is by trying to introduce germplasm from related species by interspecific hybridisation. Related species such as *B. nigra, B. juncea* and *B. campestris* have been used for this purpose but resulting plants from these crosses are frequently sterile and lose favourable characteristics which have to be regained by back crossing. This is both time consuming and laborious. The interspecific hybridisation strategy also has to cope with transferring two or more genes which are recessive in action into each of the breeding lines. Indeed, even within *B. campestris*, different genetic backgrounds have revealed different numbers of genes to be important in shatter resistance. This has necessitated breeders performing test crosses at each generation during the attempt to produce elite material. These difficulties have been compounded by the fact that shattering is a difficult and time-consuming trait to assess in the field. All these factors may account for the fact that the conventional breeding approach has made no progress over the last twenty years.

Other methods employed to try and alleviate the problem include chemicals, in the form of desiccants and pod sealants. The most widely used method to try and prevent seed loss is the mechanical technique of swathing in order to get uniform desiccation of the crop and reduce shattering by wind which occurs in the upright crop.

The present invention provides, by the use of recombinant technology, a further advantageous means for the control of pod dehiscence and/or plant abscission.

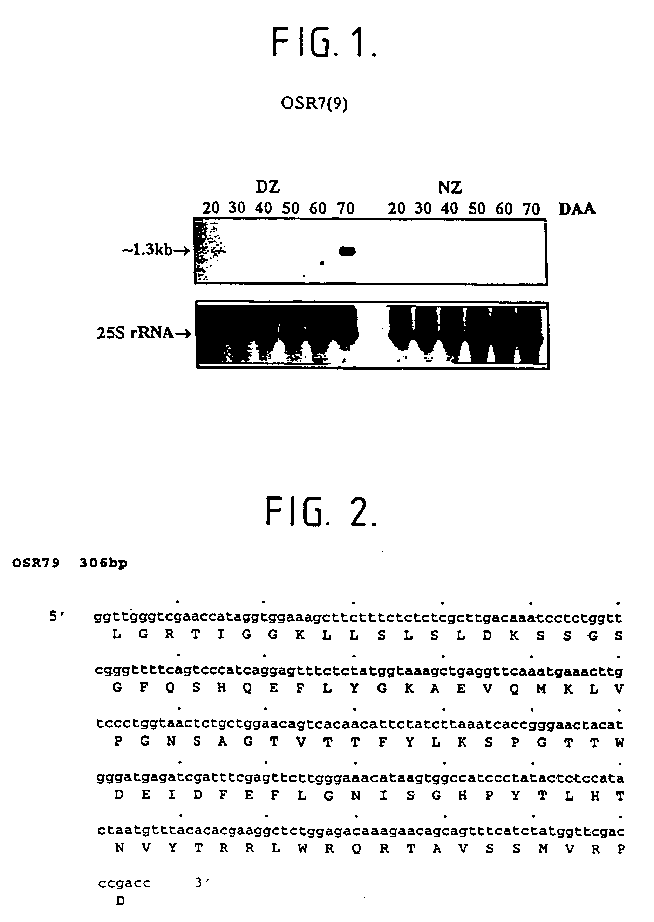

According to a first aspect of the present invention, there is provided nucleic acid, preferably a recombinant or isolated nucleic acid sequence comprising the sequence as set out in Figure 2, (here designated OSR7(9)) or a sequence substantially homologous thereto. This sequence partially encodes a protein (ie part of a protein is encoded) which affects cell wall loosening and can thus be utilised in the control of plant abscission and/or pod dehiscence. In this text, all references to sequences include the sequences themselves as well as substantial homologues, complementary sequences and substantial homologues of complementary sequences

Any substantially homologous sequence should be at least above 95% identical at the nucleic acid residue level, using the default parameters of the BLAST 2.0 program of the National Centre for Biotechnology Information (available at http:www.ncbi.nlm.nih.gov or Altschul, S. F., *et al*., Nucleic Adds Research, September 1997, 25(17), 3389-3402). Also covered by the present invention are sequences which comprise regions of complementary sequences to any sequence described above (that is any complementary sequence to the sequence shown in Figure 2, or a substantial homologue of such a sequence (as defined above)).

The nucleic acid may achieve the desired effect by it expression as antisense nucleic acid. That is, introducing the coding region, or a fragment thereof in the reverse orientation to that found in nature can result in the downregulation of the gene and hence the production of less or none of the encoded gene product. The RNA transcribed from antisense DNA is capable of binding to, and destroying the function of, a sense RNA of the sequence normally found in the cell, thereby disrupting the function. Downregularion of the protein according to the present invention can be achieved by downregulation of the gene by partial or full (transwitch) sense expression.

The recombinant or isolated nucleic acid will generally be DNA, but RNA is not excluded from the scope of the invention. The invention also relates to a nucleic acid sequence, comprising the sequence set out in Figure 2 and which encodes substantially the full length protein sequence.

Preferably the nucleic acid of the invention will include a sequence comprising a promoter or other regulatory sequence which controls its expression as desired for use in the control of plant abscission and/or dehiscence. The OSR7(9) promoter can be used (which naturally controls expression of the protein). The particular advantage of this promoter is that it can be used to drive expression of proteins in not only the pod dehiscence zone, but also in plant abcission zones. The promoter can be one which is known to be expressed preferentially in the abscission zone or the dehiscence zone. Suitable promoters include the dehiscence-zone promoter of the Sac66 gene (Jenkins *et al*., Journal of Experimental Botany 47, 111-115). Regulation of abscission can be advantageous for example in preventing fruit loss. Specifically in *B.napus* a reduction in petal abscission can reduce infection by the pathogen *Sclerotina sclerotiorum* the causative agent of stem rot. In *B. napus* stem rot leads to signaificant yield losses (HU B.C, *et al* ., (1995) Rapeseed today and tomorrow, Proceedings of the 9th International Rape Congress, Cambridge UK. pp1211-1216). The *S.sclerotiorum* acospores germinate on the *B.napus* petals, then the fungus is transferred to the stem via petal abscission. Thus there is interest in preventing petal abscission or breeding *B.napus* lines lacking petals (Hu B.C, *et al .,* (1995) Rapeseed today and tomorrow, Proceedings of the 9th International Rape Congress, Cambridge UK. pp1211-1216). The present invention also relates to nucleic acid which controls transcription and/or translation of the nucleic acid as shown in Figure 2, including ribozymes.

The invention leads to a recombinant or isolated protein or polypeptide comprising the amino acid sequence as set out in Figure 2, or a sequence substantially homologous thereto, or a fragment of the amino acid sequence set out in Figure 2. Any substantially homologous sequence should be at least above 70% identical, preferably through 75%, 80%, 85%, 87%, 90%, 95% identical or at least above 76% similar, preferably through 80%, 85%, 90%, 95% similar (all) at the amino acid residue level, using the default parameters of the BLAST 2.0 program of the National Centre for Biotechnology Information (available at http:www.ncbi.nlm.nih.gov and also at the reference cited above alongside the web-site details). Any fragment should be of 6, preferably up to 10, 15 or 20 amino acid residues in length, of such a sequence. The invention also leads to a protein sequence which comprises the amino acid sequence as set out in Figure 2 and which is substantially the complete protein.

The polypeptide /protein sequences are particularly useful in developing tools, such as antibodies having specificity for identical or substantially homologous proteins/polypeptide sequences (including orthologous sequences) for further isolation or for identification of relevant sequences.

The present invention also provides a nucleic acid sequence which encodes the amino acid sequence set out in Figure 2. The nucleic acid sequence may be the sequence set out in Figure 2 or may differ, taking into account the degenerate nucleic acid code.

At its broadest, the invention is applicable generally to plant abscission or dehiscence, that is to say to the organised shedding of a part of a plant by means of an abscission layer or dehiscence zone. Parts of plants that may from time to time be involved in abscission include leaves, petals, pods, seeds and fruit. The invention may also have application in regulating the abscission of pollen from anthers, which may be useful in generating artificially male sterile plants, which are useful for hybrid seed production (as, for example, discussed in WO-A-9211379). The invention is most useful in the control of pod dehiscence and plant shatter in plants of the Brassicaceae.

Accordingly, the present invention provides a method for regulating pod dehiscence. Nucleic acid of the invention is used to transform plant material and from such transgenic material plants are derived.
A preferred aspect of the invention is to introduce antisense nucleic acid of the invention which reduces the ultimate production of such protein/polypeptides in order to reduce or prevent dehiscence.

The present invention also provides for the use of all nucleic acid sequences described herein in the control of pod dehiscence.

In the embodiments of the invention relating to dehiscence, the invention has application to all crops that lose seed pre-harvest because of cell separation events. An economically important crop to which the invention applies is *Brassica napus*. The invention is also particularly relevant to plants that develop dry fruits, including Brassica, Synapis and other genera of the Brassicaceae, soybean and other Leguminous species and Cuphea.

In preferred embodiments of DNA sequences of this invention, 3'-transcription regulation signals, including a polyadenylation signal, may be provided. Preferred 3'-transcription regulation signals may be derived from the cauliflower mosaic virus 35S gene. It should be recognised that other 3'-transcription regulation signals could also be used. Derivation of the full length sequence of OSR7(9) as well as 3' and 5' sequences can be carried out by standard procedures well known in the art. As a general procedure, sequences 3' and 5' to the coding sequence can be identified using the following strategy: 1) use the OSR7(9) sequence (or part thereof) to probe a genomic library to obtain the full length clone, 2) locate the first ATG of the full length clone, 3) [optional] compare with other known sequences (such as on databases) to confirm the position of the ATG, 4) design primers to PCR a fragment from the ATG to a region upstream, preferably 1kb or more.

Nucleic acid (preferably recombinant DNA) in accordance with the invention may be in the form of a vector. The vector may for example be a plasmid, cosmid or phage. Vectors will frequently include one or more selectable markers to enable selection of cells transfected (or transformed: the terms are used interchangeably in this specification) with them and, preferably, to enable selection of cells harbouring vectors incorporating heterologous DNA. Appropriate start and stop signals will generally be present. Additionally, if the vector is intended for expression, sufficient regulatory sequences to drive expression will be present; however, DNA in accordance with the invention will generally be expressed in plant cells, and so microbial host expression would not be among the primary objectives of the invention, although it is not ruled out. Vectors not including regulatory sequences are useful as cloning vectors.

Cloning vectors can be introduced into *E. coli* or another suitable host which facilitate their manipulation. According to another aspect of the invention, there is therefore provided a host cell transfected or transformed with DNA as described above.

DNA in accordance with the invention can be prepared by any convenient method involving coupling together successive nucleotides, and/or ligating oligo- and/or polynucleotides, including *in vitro* processes, but recombinant DNA technology forms the method of choice.

Ultimately, DNA in accordance with the invention will where appropriate be introduced into plant cells, by any suitable means. According to a further aspect of the invention, there is provided a plant cell including DNA in accordance with the invention as described above.

Preferably, DNA is transformed into plant cells using a disarmed Ti-plasmid vector and carried by *Agrobacterium* by procedures known in the art, for example as described in

EP-A-0116718 and EP-A-0270822. Alternatively, the foreign DNA could be introduced directly into plant cells using an electrical discharge apparatus. This method is preferred where *Agrobacterium* is ineffective, for example where the recipient plant is monocotyledonous. Any other method that provides for the stable incorporation of the DNA within the nuclear DNA of any plant cell of any species would also be suitable. This includes species of plants which are not currently capable of genetic transformation.

Preferably DNA in accordance with the invention also contains a second chimeric gene (a "marker" gene) that enables a transformed plant containing the foreign DNA to be easily distinguished from other plants that do not contain the foreign DNA. Examples of such a marker gene include antibiotic resistance (Herrera-Estrella *et al., EMBO J*. **2**(6) 987-95 (1983) and Herrera-Estrella *et al., Nature* 303 209-13 (1983)), herbicide resistance (EP-A-0242246) and glucuronidase (GUS) expression (EP-A-0344029). Expression of the marker gene is preferably controlled by a second promoter which allows expression in cells other than the tapetum, thus allowing selection of cells or tissue containing the marker at any stage of regeneration of the plant. The preferred second promoter is derived from the gene which encodes the 35S subunit of Cauliflower Mosaic Virus (CaMV) coat protein. However any other suitable second promoter could be used.

A whole plant can be regenerated from a single transformed plant cell, and the invention therefore provides transgenic plants (or parts of them, such as propagating material) including DNA in accordance with the invention as described above. The regeneration can proceed by known methods.

A particular advantage of the present invention is the use of the nucleic acid sequences in the control of pod dehiscence or shatter. Identification of nucleic acid sequences involved in pod dehiscence may be unique and of particular importance. The sequence identified as OSR7(9) exhibits homology to known sequences encoding xyloglucan endotransferase (XET). XETs are implicated in cell wall loosening and are thus expected to be active in the process of cell wall separation at the pod dehiscence zone (DZ) which results in dehiscence. By reducing tianscription/translation of this enzyme at the pod DZ, one reduces, delays or prevents dehiscence such as to mitigate pod shatter.

Many of the techniques referred to herein use, or can use, hybridization to identify homologous sequences, or to probe for complementary sequences. Standard hybridization techniques can be used. For example, promoter sequences of interest can be used to identify and isolate substantially homologous promoters from other plants. Typically, a sequence is used, possibly a fragment thereof from 15 to 45 base pairs to hybridize to sequences from a suitable library under stringent conditions. Suitable conditions may be those described in Plant Genetic Transformation and Gene Expression: A Laboratory Manual, Ed. Draper, J., *et al*., 1988, Blackwell Scientific Publications, pp 252-255, modified as follows: prehybridization, hybridization and washes at 55 to 65°C, final washes (with 0.5X SSC, 0.1% SDS) omitted.

Preferred features and details of each aspect (claim category) of the invention are as for each other aspect *mutatis mutandis*.

The invention will now be illustrated by the following Examples. The Examples refer to the accompanying drawings, in which:
FIGURE 1 shows a Northern analysis of the expression of OSR7(9) in *B. napus* pods. An OSR7(9) riboprobe was generated using T3 DNA polymerase and used to probe RNA isolated from pod dehiscence zones(DZ) or from pod valves lacking DZ(NZ). The blot was reprobed with the control probe, 25S rRNA to ensure that all lanes contained RNA.
FIGURE 2 shows the DNA sequence and putative peptide encoded by OSR7(9).

### EXAMPLE 1 -

### Plant Material

Seeds of *B. napus* cv Rafal were grown as described by Meakin and Roberts, (*J. Exp. Bot*. **41**(229) 995-1002 (1990)) with the following modifications. Single seedlings were potted into 10cm pots, and after vernalization, were re-potted into 21cm pots. At anthesis tags were applied daily to record flower opening. This procedure facilitated accurate age determination of each pod. Pods were harvested at various days after anthesis (DAA). The dehiscence zone (see Figure 1) was excised from the non-zone material and seed using a scalpel blade (Meakin and Roberts *J. Exp. Bot.* 41(229) 1003-1011 (1990)) and immediately frozen in liquid N₂ and stored at -70°C.

### RNA Isolation

All chemicals were molecular biology grade and bought from either Sigma Chemical Ltd (Dorset, UK), or Fisons (Loughborough, UK). Total RNA was extracted using the polysomal extraction method of Christoffersen and Laties, *Proc. Natl. Acad. Sci.* 79 4060-4063 (1982), with the following alterations. The plant material was ground to a powder in liquid N₂ and then in 10 volumes of extraction buffer (200mM Tris-acetate [pH 8.2], 200mM magnesium acetate, 20mM potassium acetate, 20mM EDTA, 5% w/v sucrose, after sterilisation 2-mercaptoethanol was added to 15mM and cycloheximide added to a final concentration of 0.1 mg ml⁻¹). The supernatant was then layered over 8 ml 1M sucrose made with extraction buffer and centrifuged in a KONTRON^{™} (Switzerland) TFT 70.38 rotor at 45,000rpm (150,000g) for 2 hr at 2°C in a Kontron CENTRIKON^{™} T-1065 ultra-centrifuge. Pellets were then resuspended in 500µl 0.1M sodium acetate, 0.1% SDS, pH 6.0 and phenol/chloroform (1:1 v/v) extracted and the total RNA precipitated. Poly(A)⁺ RNA was isolated from total RNA extracted, from both the zone and non-zone tissue of 40, 45 and 50 DAA pods, using a Poly(A) QUIK^{™} mRNA purification kit (Stratagene, Cambridge, UK) following the manufacturers instructions, and then bulked together. Total RNA was also extracted from leaves, stems, seeds and pods using a method described by Dean *et al, EMBO J*. 4 3055-3061 (1985) for use in Northern analyses.

### Differential display

This was performed essentially as described by Liang and Pardee (1992) [Liang, P. and Pardee, A. B. (1992) Differential display of eukaryotic messenger RNA by means of the polymerase chain reaction. Science 257, 967-971] using RNA extracted from *B. napus* leaf abscission zone (AZ) and non-zone (NZ) (stem) which had been exposed to 10(L/L ethylene for 48/72 hours, with minor modifications. A targetted approach was taken in that a degenerate primer which had been designed to a conserved region of xyloglucan endotransglycosylase (XET) was used for first strand cDNA synthesis instead of the conventional oligo dT primer. First strand cDNA copies of the RNAs (AZ/NZ) were made using 50U M-MLV (Moloney Murine Leukemia Virus) reverse transcriptase (50U/(L) (Stratagene) in a 20(L reaction containing 1x M-MLV buffer, 2.5mM dNTPs (Pharmacia), 1(g RNA, 30U RNAse inhibitor (Promega) and 10(M XET2 primer (5'NGTNGGRTCRAACCANAGRTG-3' where N=A, G, C or T; R=A or G). The reaction conditions were as follows: 65°C for 5 minutes, 37°C for 90 minutes and 95°C for 5 minutes. Following first strand cDNA synthesis, 60µL dH2O were added and the samples were either used directly for PCR or stored at -20°C.

For PCR, 2µL cDNA were used as template in a 20µL reaction containing 1x PCR buffer, 1mM MgCl2, 2µM dNTPs, 10µM XET2 primer (5'NGTNGGRTCRAACCANAGRTG-3' where N=A, G, C or T; R=A or G), 2.5(M arbitrary primer A (5'-AGCCAGCGAA-3'), 0.5(L 35S-dATP (>1000 Ci/mmol) (Amersham) and 1U Taq DNA polymerase (5U/(L) (Gibco BRL). The thermocycling conditions were as follows: 40 cycles of 94°C for 30 seconds, 40°C for 2 minutes, 72°C for 30 seconds followed by 72°C for 5 minutes. The PCR products were fractionated on a 5% polyacrylamide/7M urea gel after addition of 5µL loading buffer (95% (v/v) formamide, 20mM EDTA, 0.05% (w/v) xylene cyanol, 0.05% (w/v) bromophenol blue) to each sample. Following electrophoresis the gel was dried at 80°C under vacuum for 1 hour then exposed to X-ray film (BioMax-MR, Kodak) in a light tight cassette for 48 hours. The dried gel and autoradiogram were aligned so that bands that appeared in the AZ and not in NZ could be cut out and the DNA eluted according to Liang et al. (1995) [Liang, P., Bauer, D., Averboukh, L., Warthoe, P., Rohrwild, M., Muller, H., Strauss, M. and Pardee, A. B. (1995) Analysis of altered gene expression by differential display. Methods in Enzymology 254, 304-321.]. The eluted PCR products (4µL) were reamplified in a 40µL reaction containing 1x PCR buffer, 1mM MgCl2, 20(M dNTPs, 10µM XET2 primer (5'NGTNGGRTCRAACCANAGRTG-3' where N=A, G, C or T; R=A or G), 2.5(M arbitrary primer A (5'-AGCCAGCGAA-3') and 2U Taq DNA polymerase (5U/(L) (Gibco BRL) using the following thermocycling conditions: 40 cycles of 94°C for 30 seconds, 40°C for 2 minutes, 72°C for 30 seconds followed by 72°C for 5 minutes. The resulting PCR product was cloned into the TA cloning vector (Invitrogen).

### Expression pattern and analysis of OSR7(9) in B.napus pods

Northern analysis using an antisense strand-specific riboprobe to the OSR7(9) PCR product, showed that OSR7(9) hybridised to a transcript of ~1.3kb which was expressed in the DZ of 70 DAA pods. Minimal expression was observed in the pod NZ [Figure 1]. A full length OSR7(9) cDNA can be obtained by screening of a cDNA library or using 5'RACE. Similarly, the OSR7(9) gene and promoter can be obtained by standard techniques by screening a genomic library; for example a genomic library from *B. napus* or from a relative such as *A. thaliana* can be employed.

The DNA sequence of OSR7(9) is shown in Figure 2.

### EXAMPLE 2

### Production of shatter-and abscission-resistant B.napus plants by anti-sense downregulation of OSR7(9)

The OSR7(9) promoter was obtained from an *A. thaliana* genomic library and linked to the OSR7(9) PCR product in a manner such that the PCR fragment was in the antisense oreintation. The resultant pOSR7(9)-antiORS7(9) gene was cloned into the binary vector pSCV nos nptII. SCV nos nptII is a derivative of pSCV1 (Firek *et al* ., (1993), Plant Molecular Biology 22,129-142) which contains a nos promoter driving a Kanamycin resistance gene, doned between the EcoRV and EcoRI sites of pSCVI. This binary construct was transformed into *B. napus* (var Westar) by agrobacterium transformation essentially as described in Moloney *et al*., (1989), Plant Cell Reports 8, 238-242. A proportion of the resulting *B. napus* plants were found not to express OSR7(9) and consequently to be resistant to pod shatter. These plants also exhibited a reduction in leaf and petal abscission.

### EXAMPLE 3

### Production of shatter-resistant B. napus plants by antisense downregulation of OSR7(9).

To specifically obtain pod shatter resistant plants the antisense OSR7(9) can be expressed from a promoter that is expressed in the pod DZ when OSR7(9) is expressed, but not in abscission zones. Such a promoter is that of the Sac66 gene (Jenkins *et al*., *supra*).

The Sac66 promoter was therefore obtained from a *B. napus* genomic library and linked to the OSR7(9) PCR product in a manner such that the OSR7(9) PCR fragment was in the antisense orientation. The resultant pSac66-antiOSR7(9) gene was cloned into the binary vector pSCV nos nptII. This binary construct was transformed into *B. napus* as described for example 2. A proportion of the plants were found not to express OSR7(9) and consequently to be resistant to pod shatter.

## Claims

1. A recombinant or isolated nucleic acid sequence which comprises the sequence as shown in Figure 2 or a sequence at least 95% homologous thereto or encoding the protein shown in figure 2, wherein said nucleic acid can be used to control shatter or pod dehiscence in *Brassica napus* plants.

2. A nucleic acid as claimed in claim 1, which includes a promoter or other regulatory sequence which controls expression of the sequence.

3. A recombinant or isolated nucleic acid which is antisense to a nucleic acid as defined in claim 1.

4. A nucleic acid as claimed in any one of claims 1 to 3 which is in the form of a vector.

5. A host cell transfected or transformed with the nucleic acid as claimed in any one of claims 1 to 4.

6. A plant cell including the nucleic acid as claimed in any one of claims 1 to 4.

7. A whole part or part of a plant, comprising a cell as claimed in claim 6.

8. Propagating material derived from a plant as claimed in claim 7, wherein said material includes a nucleic acid as defined in any one of claims 1 to 4.

9. A method of regulating pod dehiscence, which comprises the step of transforming propagating material from a plant with a nucleic acid sequence as defined in any one of claims 1 to 4.

10. A method as claimed in claim 9 for reducing or preventing dehiscence, wherein the nucleic acid is as defined in claim 3.

11. The use of a recombinant or isolated nucleic acid as claimed in any one of claims 1 to 4, in the control of pod dehiscence.

12. The use as claimed in claim 11 wherein the nucleic acid is used to transform a plant or plant propagating material to produce a transformed plant or plant propagating material.

## Patentansprüche

1. Rekombinante oder isolierte Nukleinsäuresequenz mit der Sequenz gemäß Abbildung 2 oder einer Sequenz, die zu mindestens 95% zu dieser Sequenz homolog ist, oder die für das Protein gemäß Abbildung 2 codiert, wobei die Nukleinsäure für die Kontrolle des Schotenplatzens bzw. der Schotendehiszenz bei *Brassica napus*-Pflanzen verwendet werden kann.

2. Nukleinsäure nach Anspruch 1, die einen Promoter oder eine sonstige Regulationssequenz, der/die die Expression der Sequenz kontrolliert, beinhaltet.

3. Rekombinante oder isolierte Nukleinsäure, die zu einer Nukleinsäure nach Anspruch 1 antisense ist.

4. Nukleinsäure nach einem der Ansprüche 1 bis 3, die in Vektorform vorliegt.

5. Wirtszelle, die mit der Nukleinsäure nach einem der Ansprüche 1 bis 4 transfiziert oder transformiert ist.

6. Pflanzenzelle, die die Nukleinsäure nach einem der Ansprüche 1 bis 4 beinhaltet.

7. Ganze Pflanze oder Teil einer Pflanze, die/der eine Zelle nach Anspruch 6 enthält.

8. Vermehrungsmaterial, das von einer Pflanze nach Anspruch 7 abstammt, wobei das Material eine Nukleinsäure nach einem der Ansprüche 1 bis 4 enthält.

9. Verfahren zur Regulation der Schotendehiszenz, das den Schritt umfaßt, daß man Vermehrungsmaterial von einer Pflanze mit einer Nukleinsäuresequenz nach einem der Ansprüche 1 bis 4 transformiert.

10. Verfahren nach Anspruch 9 zur Verringerung bzw. zum Verhindern von Dehiszenz, wobei die Nukleinsäure gemäß Anspruch 3 ist.

11. Verwendung einer rekombinanten oder isolierten Nukleinsäure nach einem der Ansprüche 1 bis 4 für die Dehiszenzkontrolle.

12. Verwendung nach Anspruch 11, wobei die Nukleinsäure für die Transformation einer Pflanze oder eines Pflanzenvermehrungsmaterials verwendet wird, um zu einer transformierten Pflanze bzw. einem transformierten Pflanzenvermehrungsmaterial zu gelangen.

## Revendications

1. Séquence d'acide nucléique recombinante ou isolée comprenant la séquence telle que présentée en figure 2, ou une séquence homologue à au moins 95 % à celle-ci ou codant pour la protéine présentée en figure 2, **caractérisée en ce que** ledit acide nucléique peut être utilisé pour contrôler l'éclatement ou la déhiscence des gousses chez des plantes de *Brassica napus*.

2. Acide nucléique selon la revendication 1, qui inclut un promoteur ou une autre séquence régulatrice qui contrôle l'expression de la séquence.

3. Acide nucléique recombinant ou isolé qui est antisens par rapport à un acide nucléique tel que défini dans la revendication 1.

4. Acide nucléique selon l'une quelconque des revendications 1 à 3, qui est sous forme d'un vecteur.

5. Cellule hôte transfectée ou transformée par l'acide nucléique selon l'une quelconque des revendications 1 à 4.

6. Cellule végétale qui inclut l'acide nucléique selon l'une quelconque des revendications 1 à 4.

7. Plante entière ou partie d'une plante comprenant une cellule selon la revendication 6.

8. Matériel de propagation provenant d'une plante selon la revendication 7, **caractérisé en ce que** ledit matériel inclut un acide nucléique tel que défini dans l'une quelconque des revendications 1 à 4.

9. Procédé de régulation de la déhiscence des gousses, qui comprend l'étape de transformation d'un matériel de propagation issu d'une plante par une séquence d'acide nucléique telle que définie dans l'une quelconque des revendications 1 à 4.

10. Procédé selon la revendication 9 destiné à réduire ou à empêcher la déhiscence, **caractérisé en ce que** l'acide nucléique est tel que défini dans la revendication 3.

11. Utilisation d'un acide nucléique recombinant ou isolé selon l'une quelconque des revendications 1 à 4, pour le contrôle de la déhiscence des gousses.

12. Utilisation selon la revendication 11, **caractérisée en ce que** l'acide nucléique est utilisé pour transformer une plante ou un matériel de propagation végétal, afin de produire une plante ou un matériel de propagation végétal transformé(e).
